# DEMANDE DE BREVET EUROPEEN

(11) **EP 4 273 163 A2**
(43) Date de publication de la demande: **08.11.2023**
(21) Numéro de dépôt: 23183322.9
(22) Date de dépôt: 05.07.2017
(51) Int. Cl.: C07K 16/00

(54) **MUTANTS FC À ACTIVITÉ FONCTIONNELLE AMELIORÉE**

(30) Priorité: 06.07.2016 FR 1656463
(62) Demande divisionnaire de: 17739229.7
(71) Demandeur: Laboratoire Français du Fractionnement et des Biotechnologies, 92800 Puteaux (FR)
(72) Inventeur: MONNET, Céline, 59130 Lambersart (FR)
(74) Mandataire: Lavoix

(57) **Abrégé**

La présente invention se rapporte à un variant d'un polypeptide parent comprenant un fragment Fc, le dit variant présentant une affinité augmentée pour au moins un des récepteurs du fragment Fc (FcR) choisi parmi les récepteurs FcyRIIIa (CD16a), FcyRIIa (CD32a), et FcyRIIb (CD32b) par rapport à celle du polypeptide parent, caractérisé en ce qu'il comprend au moins une mutation choisie parmi K290G, Y296W, V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc, la numérotation étant celle de l'index EU ou équivalent dans Kabat.

## Description

La présente invention se rapporte à un polypeptide comprenant une région Fc mutée et présentant une affinité augmentée pour au moins un des récepteurs du fragment Fc (FcR) choisi parmi les récepteurs FcyRIIIa (CD16a), FcyRIIa (CD32a), et FcyRIIb (CD32b).

Les anticorps thérapeutiques et les protéines de fusion Fc sont utilisés aujourd'hui pour traiter diverses maladies, comme la polyarthrite rhumatoïde, le psoriasis, la sclérose en plaques et de nombreuses formes de cancer. Des anticorps thérapeutiques peuvent être des anticorps monoclonaux ou polyclonaux. Les anticorps monoclonaux sont obtenus à partir d'une lignée cellulaire de production d'anticorps unique, qui montre une spécificité identique pour un seul antigène.

L'effet thérapeutique des anticorps ciblant des antigènes membranaires passe notamment par le recrutement de cellules effectrices exprimant des récepteurs pour le fragment cristallisable des anticorps (les "récepteurs Fc"). Les récepteurs Fc sont des protéines présentes à la surface de certaines cellules contribuant aux fonctions du système immunitaire, en particulier des cellules NK ("natural killer", tueuses naturelles), des macrophages, des neutrophiles et des mastocytes. Il en existe plusieurs types, qui sont classés en fonction du type d'anticorps qu'ils reconnaissent : les récepteurs Fc gamma (FcyR) se lient aux IgG, le récepteur Fc alpha (FcαR) se lie aux IgA et les récepteurs Fc epsilon (FεR) se lient aux IgE.

La liaison du récepteur Fc avec la région Fc d'un anticorps déclenche différents mécanismes en fonction de la nature de la cellule sur laquelle est exprimé ce récepteur. L'activité fonctionnelle des anticorps est notamment médiée par la liaison du fragment Fc aux récepteurs Fc. Au regard de l'importance des mécanismes liés à la liaison des récepteurs Fc aux anticorps, il serait particulièrement avantageux de disposer de variants présentant une affinité modifiée, de préférence augmentée pour les récepteurs Fc et présentant notamment une meilleure activité fonctionnelle médiée par le Fc (par exemple cytotoxicité cellulaire dépendante des anticorps, cytotoxicité dépendante du complément ou phagocytose cellulaire dépendante des anticorps).

La Demanderesse a maintenant mis au point des fragments Fc particuliers, présentant une affinité améliorée pour les récepteurs Fc, en particulier pour au moins un récepteur Fc choisi parmi les récepteurs FcyRIIIa (CD16a), FcyRIIa (CD32a), FcyRIIb (CD32b), FcyRI (CD64) et le complément C1q. Avantageusement, de tels fragments Fc possèdent une unique mutation permettant un contrôle optimal de l'impact de ladite mutation sur la liaison du fragment Fc à l'un au moins des récepteurs Fc et/ou sur ses activités effectrices. Alternativement, de tels fragments peuvent comprendre au moins une mutation additionnelle, conférant des propriétés avantageuses telles que l'augmentation de la liaison au FcRn et/ou l'augmentation de la demi-vie. Ces fragments peuvent être utilisables en thérapie, en vue d'apporter une meilleure efficacité au produit qui les contient.

### LEGENDE DES FIGURES

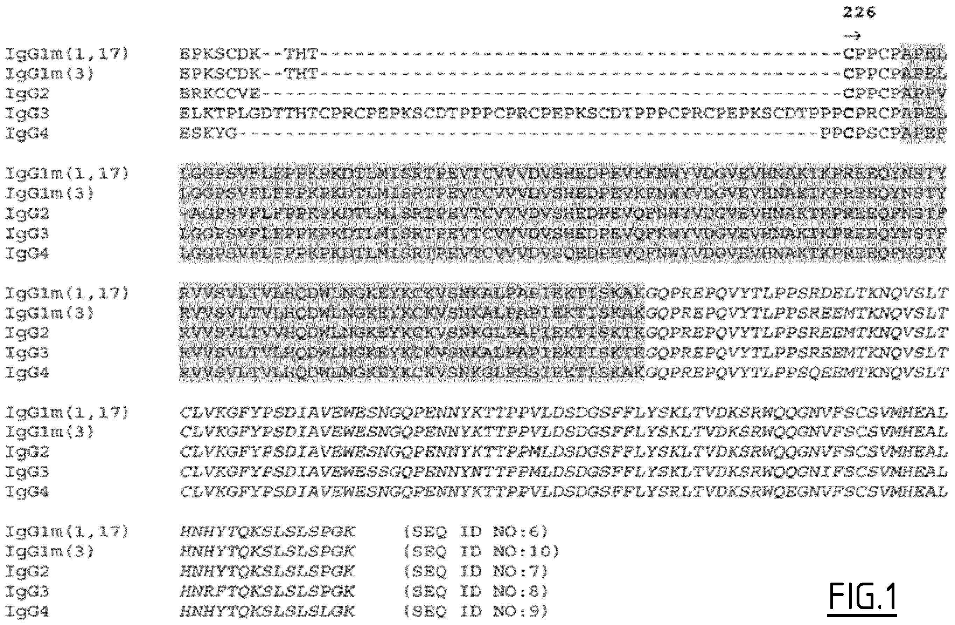

La **figure 1** montre des alignements de séquences d'IgG1 humaine natif se référant aux positions 216 à 447 (selon l'indice UE) avec les séquences correspondantes d'IgG2 humaine (SEQ ID NO: 7), IgG3 humaine (SEQ ID NO: 8) et IgG4 humaine (SEQ ID NO: 9). Les séquences d'IgG1 se réfèrent à l'allotype G1m1,17 (SEQ ID NO: 6) et à l'allotype G1m3 (SEQ ID NO: 10). Le domaine "charnière inférieure CH2-CH3" de IgG1 commence à la cystéine 226 (voir flèche). Le domaine CH2 est surligné en gris et le domaine CH3 est en italique.

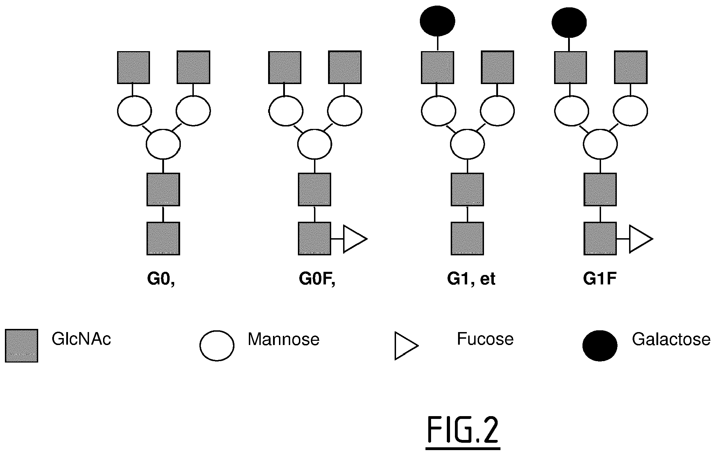

La **figure 2** montre les formes G0, G0F, G1 et G1F des structures glycanniques susceptibles d'être présentes sur les fragments Fc de l'invention.

### DESCRIPTION DE L'INVENTION

La présente invention se rapporte à un variant d'un polypeptide parent comprenant un fragment Fc, le dit variant présentant une affinité modifiée, de préférence augmentée pour au moins un des récepteurs du fragment Fc (FcR) choisi parmi les récepteurs FcyRIIIa (CD16a), FcyRIIa (CD32a), et FcyRIIb (CD32b) par rapport à celle du polypeptide parent, caractérisé en ce qu'il comprend au moins une mutation choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

Un tel variant d'un polypeptide parent est appelé "variant selon l'invention" dans la présente demande.

Tout au long de la présente demande, la numérotation des résidus dans la région Fc est celle de la chaîne lourde d'immunoglobuline selon l'index EU ou équivalent dans Kabat et al. (Sequences of Proteins of Immunological Interest, 5e éd. Public Health Service, National Institutes of Health, Bethesda, dans le Maryland, 1991). L'expression "index EU ou équivalent dans Kabat" fait référence à la numérotation EU des résidus de l'anticorps humain IgG1, IgG2, IgG3 ou IgG4. Cela est illustré sur le site IMGT (http://www.imgt.org/IMGTScientificChart/Numbering/Hu_IGHGnber.html).

Les FcR permettent aux cellules immunitaires de tirer profit de la spécificité des anticorps qui s'y fixent pour diriger leurs fonctions cellulaires sur les antigènes et les pathogènes spécifiques de cet anticorps.

Les FcyR représentent le groupe le plus diversifié de FcR et sont les principaux médiateurs des fonctions des anticorps dans l'organisme. Il existe trois familles de FcyR humains :
- FcyRI (CD64);
- FcyRII (CD32); et
- FcyRIII (CD16).

Trois d'entre eux (FcyRI, FcyRIIa, FcyRIIIa) sont des récepteurs activateurs, qui diffèrent dans leurs affinités de liaisons et dans leurs expressions cellulaires.

Le récepteur FcyRIIIa (CD16a) est impliqué dans la cytotoxicité cellulaire dépendante des anticorps (ADCC ou Antibody-Dependent Cell-mediated Cytotoxicity), il présente un polymorphisme V/F en position 158. Le récepteur FcyRIIa (CD32a) est, quant à lui, impliqué dans l'activation plaquettaire et la phagocytose ; il présente un polymorphisme H/R en position 131. Le récepteur FcyRI (CD64) est également impliqué dans la cytotoxicité cellulaire dépendante des anticorps (ADCC ou Antibody-Dependent Cell-mediated Cytotoxicity) ainsi que dans les mécanismes de phagocytose.

Enfin, le récepteur FcyRIIb (CD32b) est impliqué dans l'inhibition de l'activité cellulaire. Parmi les récepteurs Fc définis dans le cadre de l'invention, le complément C1q est impliqué dans l'activité CDC ou Complément Dépendent Cytotoxicity.

Par "polypeptide" ou "protéine", on entend une séquence comprenant au moins 100 acides aminés attachés de manière covalente.

Par "acide aminé", on entend l'un des 20 acides aminés naturels ou des analogues non naturels.

Le terme " position " signifie une position dans la séquence d'un polypeptide. Pour la région Fc, les positions sont numérotées selon l'indice de l'UE ou équivalent dans Kabat.

Le terme "anticorps" est utilisé dans le sens commun. Il correspond à un tétramère qui comprend au moins une région Fc, et deux régions variables. Les anticorps comprennent notamment les immunoglobulines pleine longueur, les anticorps monoclonaux, les anticorps multi-spécifiques, les anticorps chimériques, les anticorps humanisés et les anticorps entièrement humains. La partie amino-terminale de chaque chaîne lourde comprend une région variable d'environ 100 à 110 acides aminés responsable de la reconnaissance de l'antigène. Dans chaque région variable, trois boucles sont rassemblées pour former un site de liaison à l'antigène. Chacune des boucles est appelée une région déterminant la complémentarité (ci-après dénommé un "CDR"). La partie carboxy-terminale de chaque chaîne lourde définit une région constante principalement responsable de la fonction effectrice.

Les IgG ont plusieurs sous-classes, notamment IgG1, IgG2, IgG3 et IgG4. Les sous-classes d'IgM sont notamment IgM1 et IgM2. Ainsi, par "isotype", on entend l'une des sous-classes d'immunoglobulines définies par les caractéristiques chimiques et antigéniques de leurs régions constantes. Les isotypes connus d'immunoglobulines humaines sont les IgG1, IgG2, IgG3, IgG4, IgA1, IgA2, IgM1, IgM2, IgD et IgE.

Les IgG pleine longueur sont des tétramères et se composent de deux paires identiques de deux chaînes d'immunoglobulines, chaque paire ayant une chaîne légère et une chaîne lourde, chaque chaîne légère comprenant les domaines VL et CL, et chaque chaîne lourde comprenant les domaines VH, Cγ1 (aussi appelé CH1), Cγ2 (également appelé CH2), et Cy3 (également appelé CH3). Dans le cadre d'une IgG1 humaine, " CH1 " fait référence aux positions 118 à 215, " CH2 " renvoie aux positions 231 à 340 et " CH3 " se réfère aux positions 341 à 447 selon l'index EU ou équivalent dans Kabat. La chaîne lourde des IgG comprend également un domaine charnière flexible N-terminal qui se réfère aux positions 216 à 230 dans le cas d'IgG1. Le domaine charnière inférieur se réfère aux positions 226 à 230 selon l'index EU ou équivalent dans Kabat.

Par "région variable", on entend la région d'une immunoglobuline qui comprend un ou plusieurs domaines Ig sensiblement codés par l'un quelconque des gènes VK, Vλ et/ou VH qui composent les chaînes kappa, lambda, et lourde d'immunoglobuline, respectivement. Les régions variables comprennent les régions déterminant la complémentarité (CDR) et les régions charpentes (FR).

Le terme "Fc" ou "région Fc" désigne la région constante d'un anticorps à l'exclusion du premier domaine de région constante d'immunoglobuline (CH1). Ainsi Fc fait référence aux deux derniers domaines (CH2 et CH3) de région constante d'IgG1, et à la charnière flexible N-terminale de ces domaines. Pour une IgG1 humaine, la région Fc correspond au résidu C226 jusqu'à son extrémité carboxy-terminale, soit les résidus de la position 226 à 447, où la numérotation est selon l'index EU ou équivalent dans Kabat. La région Fc utilisée peut comprendre en outre une partie de la région charnière supérieure, située entre les positions 216 à 226 selon l'index EU ou équivalent dans Kabat ; dans ce cas, la région Fc utilisée correspond aux résidus de la position 216 à 447, 217 à 447, 218 à 447, 219 à 447, 220 à 447, 221 à 447, 222 à 447, 223 à 447, 224 à 447 ou 225 à 447, où la numérotation est selon l'index EU ou équivalent dans Kabat. De préférence dans ce cas, la région Fc utilisée correspond aux résidus de la position 216 à 447, où la numérotation est selon l'index EU ou équivalent dans Kabat. De préférence, la région Fc utilisée est choisie parmi les séquences SEQ ID NO : 1 à 10. De préférence, le fragment Fc utilisé est choisi parmi les séquences SEQ ID NO : 1, 2, 3, 4 et 5. De préférence, le fragment Fc de l'anticorps parent a pour séquence SEQ ID NO : 1. Les séquences représentées en SEQ ID NO : 1, 2, 3, 4 et 5 sont exemptes de région charnière en N-terminal. Les séquences représentées en SEQ ID NO : 6, 7, 8, 9 et 10 correspondent respectivement aux séquences représentées en SEQ ID NO : 1, 2, 3, 4 et 5 avec leurs régions charnières en N-terminal. Aussi, dans un mode de réalisation particulier, le fragment Fc de l'anticorps parent est choisi parmi les séquences SEQ ID NO : 6, 7, 8, 9 et 10. De préférence, le fragment Fc de l'anticorps parent a une séquence correspondant aux positions 1-232, 2-232, 3-232, 4-232, 5-232, 6-232, 7-232, 8-232, 9-232, 10-232 ou 11-232 de la séquence SEQ ID NO : 6.

Par "polypeptide parent", on entend un polypeptide de référence. Ledit polypeptide parent peut être d'origine naturelle ou synthétique. Dans le contexte de la présente invention, le polypeptide parent comprend une région Fc, appelée " région Fc parente ". Cette région Fc peut être choisie parmi le groupe de régions Fc de type sauvage, leurs fragments et leurs mutants. De préférence, le polypeptide parent comprend une région Fc humaine, de préférence une région Fc d'une IgG1 humaine. Le polypeptide parent peut comprendre des modifications d'acides aminés préexistantes dans la région Fc (par exemple un mutant Fc) par rapport à des régions Fc de type sauvage. Avantageusement, le polypeptide parent est une région Fc isolée (i.e. un fragment Fc en tant que tel), une séquence dérivée d'une région Fc isolée, un anticorps, une protéine de fusion comprenant une région Fc ou un conjugué Fc, cette liste n'étant pas limitative. Par " séquence dérivée d'une région Fc isolée ", on entend une séquence comprenant au moins deux régions Fc isolées liées entre elles, telle qu'un scFc (single chain Fc) ou un multimère Fc. Par " protéine de fusion comprenant une région Fc ", on entend une séquence polypeptidique fusionnée à une région Fc, ladite séquence polypeptidique étant de préférence choisie parmi les régions variables de tout anticorps, les séquences de liaison d'un récepteur à son ligand, les molécules d'adhésion, les ligands, les enzymes, les cytokines et les chimiokines. Par " conjugué Fc ", on entend un composé qui est le résultat du couplage chimique d'une région Fc avec un partenaire de conjugaison. Le partenaire de conjugaison peut être protéique ou non protéique. La réaction de couplage utilise généralement des groupes fonctionnels sur la région Fc et le partenaire de conjugaison. Divers groupes de liaison sont connus dans l'art antérieur comme étant appropriés pour la synthèse d'un conjugué; par exemple, des groupes de liaison homo-ou hétérobifonctionnels sont bien connus (voir, catalogue Pierce Chemical Company, 2005-2006, section technique sur des agents de réticulation, pages 321-350). Parmi les partenaires de conjugaison appropriés, on peut citer les protéines thérapeutiques, les étiquettes, les agents cytotoxiques tels que les agents chimiothérapeutiques, les toxines et leurs fragments actifs. Avantageusement, le polypeptide parent - et donc le variant selon l'invention - consiste en une région Fc.

Avantageusement, le polypeptide parent - et donc le variant selon l'invention - est un anticorps.

Enfin, de préférence, le polypeptide parent - et donc le variant selon l'invention - est un polypeptide produit dans le lait d'animaux transgéniques.

Par " mutation ", on entend un changement d'au moins un acide aminé de la séquence d'un polypeptide, notamment un changement d'au moins un acide aminé de la région Fc du polypeptide parent. Le polypeptide muté ainsi obtenu est un polypeptide variant ; c'est un variant selon l'invention. Un tel polypeptide comprend une région Fc mutée, par rapport au polypeptide parent. De préférence, la mutation est une substitution, une insertion ou une délétion d'au moins un acide aminé. Par "substitution", on entend le remplacement d'un acide aminé à une position particulière dans une séquence de polypeptide parent par un autre acide aminé. Par exemple, la substitution N434S se réfère à un polypeptide variant, en l'occurrence un variant pour lequel l'asparagine à la position 434 est remplacé par la sérine. Par "insertion d'acide aminé" ou "insertion", on entend l'addition d'un acide aminé à une position particulière dans une séquence de polypeptide parent. Par exemple, l'insertion G>235-236 désigne une insertion de glycine entre les positions 235 et 236. Par " délétion d'acides aminés" ou "délétion", on entend la suppression d'un acide aminé à une position particulière dans une séquence de polypeptide parent. Par exemple, E294del désigne la suppression de l'acide glutamique en position 294. De préférence, le libellé suivant de mutation est utilisé: " 434S " ou " N434S ", et signifie que le polypeptide parent comprend l'asparagine en position 434, qui est remplacée par la sérine dans le variant. Dans le cas d'une combinaison de substitutions, le format préféré est le suivant: " 2591/315D/434Y " ou " V259I/N315D/N434Y ". Cela signifie qu'il existe trois substitutions dans le variant, en positions 259, 315 et 434, et que l'acide aminé en position 259 du polypeptide parent, à savoir la valine, est remplacé par l'isoleucine, que l'acide aminé en position 315 du polypeptide parent, soit l'asparagine, est remplacé par l'acide aspartique et que l'acide aminé en position 434 du polypeptide parent, soit l'asparagine, est remplacé par la tyrosine.

Le variant selon l'invention présente une activité fonctionnelle médiée par la région Fc modifiée, de préférence augmentée par rapport à celle du polypeptide parent.

Par "activité fonctionnelle médiée par la région Fc", on entend notamment les fonctions effectrices. L'activité fonctionnelle médiée par la région Fc comprend ainsi notamment la cytotoxicité cellulaire dépendante des anticorps (ADCC ou Antibody-Dependent Cell-mediated Cytotoxicity), la cytotoxicité dépendante du complément (CDC ou Complément Dependent Cytotoxicity), la phagocytose cellulaire dépendante des anticorps (ADCP), l'activité d'endocytose, la sécrétion de cytokines, ou une combinaison d'au moins deux de ces activités. De préférence, l'activité fonctionnelle médiée par la région Fc considérée dans l'invention est choisie parmi l'ADCC, l'ADCP, la CDC et leur combinaison. Cette activité fonctionnelle peut être évaluée par des procédés bien connus de l'art antérieur. L'activité fonctionnelle médiée par la région Fc du variant selon l'invention est augmentée par rapport à celle du polypeptide parent, typiquement d'un ratio au moins égal à 2, de préférence supérieur à 5, de préférence supérieur à 10, de préférence supérieur à 15, de préférence supérieur à 20, de préférence supérieur à 25, et de préférence supérieur à 30.

Préférentiellement, ladite région Fc mutée a une affinité modifiée, préférentiellement augmentée, pour au moins l'un des FcR. Préférentiellement, l'affinité est augmentée, par rapport à celle du Fc parent, d'un ratio au moins égal à 2, de préférence supérieur à 5, de préférence supérieur à 10, de préférence supérieur à 15, de préférence supérieur à 20, de préférence supérieur à 25, et de préférence supérieur à 30. En d'autres termes, l'affinité de la région Fc mutée pour un FcR est supérieure à celle du polypeptide parent.

L'affinité d'un polypeptide comprenant une région Fc pour un FcR peut être évaluée par des procédés bien connus de l'art antérieur. Par exemple, l'homme de l'art peut déterminer l'affinité (Kd) en utilisant la résonance plasmonique de surface (SPR). Alternativement, l'homme de l'art peut effectuer un test ELISA approprié. Un dosage ELISA approprié permet de comparer les forces de liaison du Fc parent et du Fc muté. Les signaux détectés spécifiques du Fc muté et du Fc parent sont comparés. L'affinité de liaison peut être indifféremment déterminée en évaluant les polypeptides entiers ou en évaluant les régions Fc isolées de ceux-ci.

De préférence, la région Fc mutée du variant selon l'invention comprend de 1 à 20 mutations par rapport au polypeptide parent, de préférence de 2 à 20 mutations. Par "de 1 à 20 modifications d'acides aminés", on englobe 1, 2, 3, 4, 5, 6, 7, 8, 9, 10, 11, 12, 13, 14, 15, 16, 17, 18, 19 et 20 mutations d'acides aminés. De préférence, elle comprend de 1 à 15 mutations, de préférence de 2 à 15 mutations, de préférence de 1 à 10 mutations par rapport au polypeptide parent, de préférence de 2 à 10 mutations.

De préférence, le variant selon l'invention est caractérisé en ce que la mutation est choisie parmi une insertion, une substitution, de préférence ponctuelle, et une délétion.

De préférence, le variant selon l'invention comprend au moins une mutation i) choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

Dans un mode de réalisation particulier, l'invention se rapporte à un variant d'un polypeptide parent comprenant un fragment Fc, le dit variant présentant une affinité modifiée, de préférence augmentée pour au moins un des récepteurs du fragment Fc (FcR) choisi parmi les récepteurs FcyRIIIa (CD16a), FcyRIIa (CD32a), et FcyRIIb (CD32b) par rapport à celle du polypeptide parent, caractérisé en ce qu'il comprend une unique mutation i) choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

Ainsi, dans un mode de réalisation particulier, le variant selon l'invention comprend une unique mutation i) choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

Dans un autre mode de réalisation particulier, le dit variant Fc comprend au moins deux mutations i), lesdites mutations étant choisies parmi i) V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat et avec la condition que les mutations ne soient pas identiques.

Dans un mode de réalisation plus particulier, le dit variant Fc comprend au moins trois mutations i), lesdites mutations i) étant choisies parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat et avec la condition que les mutations ne soient pas identiques.

Dans un mode de réalisation plus particulier, le dit variant Fc comprend au moins quatre mutations i), lesdites mutations i) étant choisies parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat et avec la condition que les mutations ne soient pas identiques.

Dans un mode de réalisation, le variant selon l'invention présente une affinité augmentée pour le récepteur FcyRIIIa (CD16a). Dans ce mode de réalisation particulier, ledit variant comprend au moins une mutation i) choisie parmi S298A, S298R, F243S, F243L, L242A, L242F, L242G, L242I, L242K, L242S, L242V, V240I, V240M, V240N, V240S, E258I, T260A, K290D, K290E, K290G, K290H, K290Q, K290S, K290Y, Y296H, Y296W dudit fragment Fc;
la numérotation étant celle de l'index EU ou équivalent dans Kabat.

Dans un autre mode de réalisation, le variant selon l'invention présente une affinité augmentée pour le récepteur FcyRIIa (CD32a). Dans ce mode de réalisation particulier, ledit variant comprend au moins une mutation i) choisie parmi F241H, F241Y, F243L, L242A, L242F, L242G, L242H, L242I, L242K, L242P, L242S, L242T, L242V, V240H, V240I, V240M, V240S, E258G, E258I, E258R, E258M, E258Q, E258Y, S267A, S267Q, S267V, T260A, T260H, T260I, T260M, T260N, T260R, T260S, T260W, V259C, V259I, V259L, V262A, V262S, V263T, V264L, V264S, V264T, V266L, V266M, E293A, E293D, E293G, E293M, E293Q, E293S, E293T, E294A, E294G, E294P, E294Q, E294R, E294T, E294V, K290D, K290E, K290G, K290H, K290L, K290N, K290Q, K290R, K290S, K290Y, P291G, P291Q, P291R, Q295I, Q295M, R292I, R292L, R301A, R301P, R301S, S304T, V302A, V302F, V302L, V302M, V302R, V302S, V303Y, V305A, V305F, V305L, V305R, V305S, Y300I, Y300Vou Y300W ; la numérotation étant celle de l'index EU ou équivalent dans Kabat. Dans un autre mode de réalisation, le variant selon l'invention présente une affinité augmentée pour le récepteur FcyRIIb (CD32b). Dans ce mode de réalisation particulier, ledit variant comprend au moins une mutation i) choisie parmi E258R, E258Y, V262A, S267A, S267Q, S267V, V264S, V266L, V266M, K290R, R301A, R301M, S304T, V302A, V302L, V302R, V303S, V305A, V305F, V305I, V305R, Y300V dudit fragment Fc ; la numérotation étant celle de l'index EU ou équivalent dans Kabat.

De préférence, le variant selon l'invention est caractérisé en ce que le fragment Fc du polypeptide parent comprend déjà au moins :
(ii) une mutation choisie parmi 378V, 378T, 434Y et 434S; et
(iii) au moins une mutation choisie parmi 226G, P228L, P228R, 230S, 230T, 230L, 241L, 264E, 307P, 315D, 330V, 362R, 378V, 378T, 389T, 389K, 434Y et 434S,
la numérotation étant celle de l'index EU ou équivalent dans Kabat et avec la condition que les mutations (ii) et (iii) n'aient pas lieu sur les mêmes acides aminés.

Ainsi selon un aspect particulier, l'invention se rapporte à un variant d'un polypeptide parent comprenant un fragment Fc, le dit variant présentant une affinité modifiée, de préférence augmentée, pour au moins un des récepteurs du fragment Fc (FcR) choisi parmi les récepteurs FcyRIIIa (CD 16a), FcyRIIa (CD32a), et FcyRIIb (CD32b) par rapport à celle du polypeptide parent, caractérisé en ce qu'il comprend au moins une mutation i) choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; et comprenant en outre au moins:
(ii) une mutation choisie parmi 378V, 378T, 434Y et 434S; et
(iii) au moins une mutation choisie parmi 226G, P228L, P228R, 230S, 230T, 230L, 241L, 264E, 307P, 315D, 330V, 362R, 378V, 378T, 389T, 389K, 434Y et 434S, avec la condition que les mutations (ii) et (iii) n'aient pas lieu sur les mêmes acides aminés,
la numérotation étant celle de l'index EU ou équivalent dans Kabat,

Dans un mode de réalisation particulier, ledit variant comprend au moins une combinaison de mutations choisie dans le groupe constitué de 226G/315D/434Y, 230S/315D/434Y, 230T/315D/434Y, 230T/264E/434S, 230T/389T/434S, 241L/264E/378V, 241L/264E/434S, 250A/389K/434Y, 259I/315D/434Y, 264E/378T/396L, 264E/378V/416K, 264E/378V/434S, 264E/396L/434S, 294del/307P/434Y, 307P/378V/434Y, 315D/330V/434Y, 315D/382V/434Y et 378V/383N/434Y,
étant entendu que la numération des positions des acides aminés du fragment Fc est celle de l'index EU ou équivalent dans Kabat.

Dans un mode de réalisation particulier, ledit variant comprend en outre au moins une mutation choisie dans le groupe constitué de 226G, 227L, 230S, 230T, 230L, 231T, 241L, 243L, 250A, 256N, 259I, 264E, 265G, 267R, 290E, 294del, 303A, 305A, 307P, 307A, 308I, 315D, 322R, 325S, 327V, 330V, 342R, 347R, 352S, 361D, 362R, 362E, 370R, 378V, 378T, 382V, 383N, 386R, 386K, 387T, 389T, 389K, 392R, 395A, 396L, 397M, 403T, 404L, 415N, 416K, 421T, 426T, 428L, 433R, 434Y, 434S et 439R,
étant entendu que la numération des positions des acides aminés du fragment Fc est celle de l'index EU ou équivalent dans Kabat.

Dans un mode de réalisation particulier, ledit variant comprend au moins une combinaison de mutations choisie dans le groupe constitué de 307A/315D/330V/382V/389T/434Y, 256N/378V/383N/434Y, 315D/330V/361D/378V/434Y, 259I/315D/434Y, 230S/315D/428L/434Y, 241L/264E/307P/378V/433R, 250A/389K/434Y, 305A/315D/330V/395A/434Y, 264E/386R/396L/434S/439R, 315D/330V/362R/434Y, 294del/307P/434Y, 305A/315D/330V/389K/434Y, 315D/327V/330V/397M/434Y, 230T/241L/264E/265G/378V/421T, 264E/396L/415N/434S, 227L/264E/378V/434S, 264E/378T/396L, 230T/315D/362R/426T/434Y, 226G/315D/330V/434Y, 230L/241L/243L/264E/307P/378V, 250A/315D/325S/330V/434Y, 290E/315D/342R/382V/434Y, 241L/315D/330V/392R/434Y, 241L/264E/307P/378V/434S, 230T/264E/403T/434S, 264E/378V/416K, 230T/315D/362E/434Y, 226G/315D/434Y, 226G/315D/362R/434Y, 226G/264E/347R/370R/378V/434S, 3081/315D/330V/382V/434Y, 230T/264E/378V/434S, 231T/241L/264E/378T/397M/434S, 230L/264E/378V/434S, 230T/315D/330V/386K/434Y, 226G/315D/330V/389T/434Y, 267R/307P/378V/421T/434Y, 230S/315D/387T/434Y, 230S/264E/352S/378V/434S et 230T/303A/322R/389T/404L/434S, étant entendu que la numération des positions des acides aminés du fragment Fc est celle de l'index EU ou équivalent dans Kabat.

Dans un mode de réalisation particulier, le variant selon l'invention comprend au moins une mutation i), de préférence une unique mutation i), choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; et une combinaison de mutations ii) et iii) choisie parmi 315D/330V/361D/378V/434Y, 230S/315D/428L/434Y, 307A/315D/330V/382V/389T/434Y, 259I/315D/434Y, 256N/378V/383N/434Y.

De préférence, le variant selon l'invention est caractérisé en ce que le fragment Fc du polypeptide parent comprend déjà au moins :
(iv) une mutation choisie parmi 307N, 326E, 326T, 334N, 334R, 352L, 378V, 378T, 394P, 396L, 397M, 421T ; et
(v) au moins une mutation choisie parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N,
la numérotation étant celle de l'index EU ou équivalent dans Kabat et avec la condition que les mutations (iv) et (v) n'aient pas lieu sur les mêmes acides aminés.

Ainsi selon un aspect particulier, l'invention se rapporte à un variant d'un polypeptide parent comprenant un fragment Fc, le dit variant présentant une affinité modifiée, de préférence augmentée, pour au moins un des récepteurs du fragment Fc (FcR) choisi parmi les récepteurs FcyRIIIa (CD16a), FcyRIIa (CD32a), et FcyRIIb (CD32b) par rapport à celle du polypeptide parent, caractérisé en ce qu'il comprend au moins une mutation i) choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; et comprenant en outre au moins:
(iv) une mutation choisie parmi 307N, 326E, 326T, 334N, 334R, 352L, 378V, 378T, 394P, 396L, 397M, 421T ; et
(v) au moins une mutation choisie parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N,
la numérotation étant celle de l'index EU ou équivalent dans Kabat et avec la condition que les mutations (i), (iv) et (v), n'aient pas lieu sur les mêmes acides aminés.

De préférence, la mutation (iv) est choisie parmi 378V, 396L et 397M. De préférence, le polypeptide comprend en outre une mutation choisie parmi 248E, 326T, 333G et 423Y.

De préférence, la mutation (v) selon l'invention est choisie parmi 226Y, 227S, 230S, 231V, 234P, 243I, 243L, 246R, 246E, 247T, 248E, 253F, 254F, 255W, 259A, 261R, 262A, 263A, 266M, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378T, 379A, 380G, 383R, 385R, 389S, 389T, 392R, 393A, 393I, 394P, 396L, 397I, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N.

Dans un mode de réalisation, la mutation (iv) est choisie parmi 378V, 396L et 397M et la mutation (v) est choisie parmi 248E, 316D, 326E, 333G, 378T, 396L et 421T.

Dans un autre mode de réalisation, la mutation (iv) est 378Vet la mutation (v) est choisie parmi 298N et 336T.

Dans un autre mode de réalisation, la mutation (iv) est choisie parmi 378V, 396L et 397M; et la mutation (v) est choisie parmi 231V, 286I, 286Y, 290E, 315D, 334N, 352S, 361H, 366A, 378T, 397M, 412M, 421T et 423Y.

Dans un autre mode de réalisation, la mutation (iv) est 378V; et la mutation (v) est choisie parmi 248E, 308A, 334R, 447N.

Dans un autre mode de réalisation, la mutation (iv) est choisie parmi 378V, 326E, 397M, 334N et 396L ; et la mutation (v) est choisie parmi 316D, 397M, 334N, 248E, 231V, 246R, 336T, 421T, 361H, 366A, 439R, 290E, 394P, 307P, 378V, 378T, 286I, 286Y et 298N.

Dans un autre mode de réalisation, la mutation (iv) est choisie parmi 378V, 326E, 397M, 307N, 394P, 326T, 396L et 334N ; et la mutation (v) est choisie parmi 316D, 334R, 334N, 323I, 231V, 246R, 336T, 378T, 286Y, 286I, 352S, 383R, 359A, 421T, 361H, 315D, 366A, 290E, 307P et 439R. De préférence, la mutation (v) est choisie parmi : 316D, 334R, 334N, 323I, 231V, 246R, 336T, 378T, 286Y, 286I, 352S, 383R, 359A, 421T, 361H, 366A, 290E, 307P et 439R.

Dans un autre mode de réalisation, la mutation (iv) est choisie parmi 326E, 326T, 378V, 397M, 352L, 394P, 396L et 421T; et la mutation (v) est choisie parmi 316D, 334R, 248E, 334N, 418P, 231V, 320E, 402D, 359A, 383R, 421T et 361H.

Dans un autre mode de réalisation, la mutation (iv) est choisie parmi 378V, 378T, 396L, 421T, 334R et 326E; et la mutation (v) est choisie parmi 361H, 290E, 316D, 248E, 410R, 421T, 334R, 394P, 307P, 447N, 378V, 284L, 421T, 396L, 286I, 315D et 397M.

Dans un autre mode de réalisation, la mutation (iv) est choisie parmi 378V, 326E, 397M, 334N et 396L ; et la mutation (v) est choisie parmi 316D, 397M, 334N, 248E, 231V, 246R, 336T, 421T, 361H, 366A, 439R, 290E, 394P, 307P, 378V, 378T, 286I, 286Y et 298N.

Dans un autre mode de réalisation, la mutation (iv) est choisie parmi 326E, 326T, 352L, 378V, 378T, 396L, 397M, 421T, 334N, 334R, 307N et 394P , et la mutation (v) consiste en au moins 2 mutations choisies parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N.

De préférence, les au moins 2 mutations (v) sont choisies parmi 226Y, 227S, 230S, 231V, 234P, 243I, 243L, 246R, 246E, 247T, 248E, 253F, 254F, 255W, 259A, 261R, 262A, 263A, 266M, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378T, 379A, 380G, 383R, 385R, 389S, 389T, 392R, 393A, 393I, 394P, 396L, 397I, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N.

De préférence, la région Fc mutée du polypeptide selon l'invention comprend une combinaison de mutations choisie parmi les combinaisons :
K320E/T394P/G402D;
K290E/K320E/T350A/P396L;
T359A/S383R/V397M.

Selon un autre aspect de l'invention, on utilise une composition comprenant une pluralité de variants d'un polypeptide parent comprenant un fragment Fc, qui possèdent tous substantiellement la même séquence, lesdits variants comprenant des fragments Fc qui, pris dans leur ensemble, présentent un profil de glycosylation particulier.

Selon un aspect particulier, les fragments Fc des variants au sein d'une composition utilisée dans le cadre de l'invention possèdent sur leur site de glycosylation (Asn 297, la numérotation étant celle de l'index EU ou équivalent dans Kabat) des N-glycannes, caractérisés en ce que lesdits N-glycannes des fragments Fc présentent un taux de fucosylation inférieur à 65%, de préférence inférieur à 60%, de préférence inférieur à 55%, de préférence inférieur à 50%, de préférence inférieur à 45%, de préférence inférieur à 40%, de préférence inférieur à 35%, de préférence inférieur à 30%, de préférence inférieur à 25%, de préférence inférieur à 20%. Selon un autre aspect encore, les fragments Fc des variants au sein d'une composition utilisée dans le cadre de l'invention possèdent sur leur site de glycosylation (Asn 297) des N-glycannes, caractérisés en ce que lesdits N-glycannes des fragments Fc présentent une structure glycannique de type biantenné, avec des chaînes courtes, une faible sialylation, présentant des N-acétylglucosamines terminaux non intercalaires.

Selon un aspect plus particulier, les fragments Fc des variants au sein d'une composition utilisée dans le cadre de l'invention possèdent sur leur site de glycosylation (Asn 297) des N-glycannes, caractérisés en ce que lesdits N-glycannes des fragments Fc présentent une teneur supérieure à 60 % pour les formes G0+G1+G0F+G1F, les formes G0F+G1F étant inférieures à 50 %.

Selon un autre aspect plus particulier, les fragments Fc des variants au sein d'une composition utilisée dans le cadre de l'invention possèdent sur leur site de glycosylation (Asn 297) des N-glycannes, caractérisés en ce que lesdits N-glycannes des fragments Fc présentent une teneur supérieure à 60 % pour les formes G0+G1+G0F+G1F, la teneur en fucose étant inférieure à 65 %.

Selon un autre aspect encore plus particulier, les fragments Fc des variants au sein d'une composition utilisée dans le cadre de l'invention possèdent sur leur site de glycosylation (Asn 297) des N-glycannes, caractérisés en ce que lesdits N-glycannes des fragments Fc présentent une teneur inférieure à 40% pour les formes G1F+G0F.

Selon un aspect plus particulier, les fragments Fc des variants au sein d'une composition utilisée dans le cadre de l'invention possèdent sur leur site de glycosylation (Asn 297) des N-glycannes, lesdits N-glycannes des fragments Fc présentant un taux de fucosylation égal à 0%. L'invention fournit ainsi une composition comprenant des variants d'un polypeptide parent comprenant un fragment Fc, les fragments Fc desdits variants possédant sur le site de glycosylation Asn297 des N-glycannes caractérisés en ce que lesdits N-glycannes des fragments Fc sont dépourvus de fucose.

Aussi, selon un aspect particulier, les fragments Fc des variants au sein d'une composition utilisée dans le cadre de l'invention possèdent sur le site de glycosylation Asn297 des N-glycannes, caractérisés en ce que lesdits N-glycannes des fragments Fc présentent un taux de fucosylation compris entre 20 % et 55%. En particulier, l'invention fournit une composition comprenant des variants d'un polypeptide parent comprenant un fragment Fc, les fragments Fc desdits variants possédant sur le site de glycosylation Asn297 des N-glycannes caractérisée en ce que lesdits N-glycannes des fragments Fc présentent un taux de fucosylation compris entre 20 % et 50%, entre 25% et 55%, entre 25% et 50%, entre 20% et 45% ou entre 25 et 45%.

Selon un aspect plus particulier, une composition utile selon l'invention comprend des variants d'un polypeptide parent comprenant un fragment Fc, les fragments Fc desdits variants possédant sur le site de glycosylation Asn297 des N-glycannes, caractérisés en ce que lesdits N-glycannes des fragments Fc présentent une teneur supérieure à 60 %, de préférence supérieure à 80%, pour les formes G0+G1+G0F+G1F, les formes G0F+G1F étant inférieures à 50 %, de préférence inférieure à 40%, ou à 30%.

Selon un autre aspect plus particulier, les N-glycannes des fragments Fc au sein de la composition présentent une teneur supérieure à 60 % pour les formes G0+G1+G0F+G1F, la teneur en fucose étant inférieure à 65 %.

Selon encore un autre aspect plus particulier, les N-glycannes des fragments Fc au sein de la composition présentent une teneur inférieure à 50% pour les formes G1F+G0F, de préférence inférieure à 40%, ou à 30%.

Les formes G0, G0F, G1 et G1F sont sélectionnées parmi les formes indiquées sur la Figure 2.

De manière avantageuse, les N-glycannes des fragments Fc au sein de la composition de variants ont une teneur moyenne en acide sialique inférieure à 25%, 20%, 15%, ou 10%, de préférence 5%, 4% 3% ou 2%.

Une composition qui peut être utilisée dans le cadre de l'invention comprend des variants d'un polypeptide parent comprenant un fragment Fc, les fragments Fc desdits variants possédant sur leur site de glycosylation (Asn 297) des N-glycannes, lesdits N-glycannes des fragments Fc présentant une structure glycannique de type biantenné, avec des chaînes courtes, une faible sialylation, et une faible fucosylation, les N-glycannes ayant par exemple une teneur supérieure à 60% pour les formes G0 + G1 + G0F + G1F, et une fucosylation inférieure à 60%, de préférence inférieure à 55%, les N-glycannes ayant par exemple une teneur inférieure à 50% de formes G0F + G1F et une fucosylation inférieure à 55%.

Dans un mode de réalisation particulier, les fragments Fc selon l'invention présentent des structures glycaniques telles que décrites dans la demande de brevet WO01/77181.

Selon un mode de réalisation avantageux, les fragments Fc utilisés dans l'invention comprennent au moins une mutation d'un acide aminé par rapport à un fragment Fc parent, et possèdent sur leur site de glycosylation (Asn 297) des N-glycannes, lesdits N-glycannes des fragments Fc présentant un taux de fucosylation inférieur à 65%, de préférence inférieur à 60%, de préférence inférieur à 55%, de préférence inférieur à 50%, de préférence inférieur à 45%, de préférence inférieur à 40%, de préférence inférieur à 35%, de préférence inférieur à 30%, de préférence inférieur à 25%, de préférence inférieur à 20%. De préférence, les fragments Fc portent au moins une mutation i) choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat, et possèdent en outre sur leur site de glycosylation (Asn 297) des N-glycannes, présentant un taux de fucosylation inférieur à 55%, de préférence inférieur à 50%, de préférence inférieur à 45%, de préférence inférieur à 40%, de préférence inférieur à 35%, de préférence inférieur à 30%, de préférence inférieur à 25%, de préférence inférieur à 20%.

De préférence encore, les fragments Fc de la composition selon l'invention portent une mutation i) choisie parmi V240H, F241H, F241Y, L242H, L242P, L242T, E258G, E258R, E258M, E258Q, E258Y, V259C, V259I, V259L, T260H, T260I, T260M, T260N, T260R, T260S, T260W, V262A, V262S, V263T, V264L, V264S, V264T, V266L, V266M, S267A, S267Q, S267V, K290L,K290N, K290R, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S, dudit fragment Fc; la numérotation étant celle de l'index EU ou équivalent dans Kabat, et possèdent en outre sur leur site de glycosylation (Asn 297) des N-glycannes, présentant un taux de fucosylation inférieur à 55%, de préférence inférieur à 50%, de préférence inférieur à 45%, de préférence inférieur à 40%, de préférence inférieur à 35%, de préférence inférieur à 30%, de préférence inférieur à 25%, de préférence inférieur à 20%.

De façon avantageuse, les fragments Fc ayant une glycosylation modifiée au niveau du site de glycosylation en position 297, en particulier une faible fucosylation, présente une augmentation de la liaison dudit fragment aux récepteurs Fcgamma (FcyR), en particulier le récepteur FcyRIIIa (CD16a).

De préférence, lesdits fragments Fc ont une affinité pour le CD16a au moins égale à 2×10⁶ M⁻¹, au moins égale à 2×10⁷ M⁻¹, 2×10⁸ M⁻¹ ou 2×10⁹ M⁻¹, telle que déterminée par l'analyse Scatchard ou la technologie BIAcore (Label-free surface plasmon résonance based technology).

De préférence, le variant selon l'invention est caractérisé en ce qu'il comprend de 1 à 20 mutations dudit fragment Fc, de préférence de 1 à 10 mutations.

De préférence, le variant selon l'invention est caractérisé en ce que le polypeptide parent comprend un fragment Fc parent qui est un fragment Fc humain, de préférence un fragment Fc d'une IgG1 humaine ou d'une IgG2 humaine.

De préférence, le variant selon l'invention est caractérisé en ce qu'il est choisi parmi un fragment Fc isolé, une séquence dérivée d'un fragment Fc isolé, un anticorps et une protéine de fusion comprenant un fragment Fc.

De préférence, le variant selon l'invention est caractérisé en ce qu'il est un anticorps.

La présente invention a également pour objet une composition de polypeptides selon l'invention.

La présente invention a également pour objet une composition pharmaceutique comprenant (1) un variant selon l'invention ou une composition telle que décrite au paragraphe précédent, et (2) au moins un excipient pharmaceutiquement acceptable.

La présente invention a également pour objet le variant selon l'invention ou la composition telle que décrite précédemment, pour son utilisation comme médicament.

Comme indiqué précédemment, avantageusement, le polypeptide parent - et donc le variant selon l'invention - est un anticorps. Dans ce cas, l'anticorps peut être dirigé contre un antigène choisi parmi un antigène tumoral, un antigène viral, un antigène bactérien, un antigène fongique, une toxine, une cytokine membranaire ou circulante et un récepteur membranaire.

Lorsque l'anticorps est dirigé contre un antigène tumoral, son utilisation convient particulièrement dans le traitement de cancers. Par " cancer ", on entend toute condition physiologique caractérisée par une prolifération anormale des cellules. Des exemples de cancers incluent notamment les carcinomes, les lymphomes, les blastomes, les sarcomes (incluant les liposarcomes), les tumeurs neuroendocrines, les mésothéliomes, les méningiomes, les adénocarcinomes, les mélanomes, les leucémies et les pathologies lymphoïdes malignes, cette liste n'étant pas exhaustive.

Lorsque l'anticorps est dirigé contre un antigène viral, son utilisation convient particulièrement dans le traitement des infections virales. Les infections virales sont notamment des infections dues au VIH, à un rétrovirus, à un virus Coxsackie, au virus de la variole, de la grippe, de la fièvre jaune, du Nil occidental, à un cytomégalovirus, à un rotavirus ou au virus de l'hépatite B ou C, cette liste n'étant pas exhaustive.

Lorsque l'anticorps est dirigé contre une toxine, son utilisation convient particulièrement dans le traitement des infections bactériennes, par exemple les infections par la toxine tétanique, la toxine diphtérique, les toxines du charbon *Bacillus anthracis,* ou encore dans le traitement des infections par les toxines botuliques, les toxines de la ricine, les shigatoxines, cette liste n'étant pas exhaustive.

Lorsque l'anticorps est dirigé contre une cytokine, son utilisation convient particulièrement dans le traitement des maladies inflammatoires et/ou auto-immunes. Les maladies inflammatoires et/ou auto-immunes incluent notamment le purpura thrombotique thrombocytopénique (PTI), le rejet de greffes ou d'organes, la maladie du greffon contre l'hôte, la polyarthrite rhumatoïde, le lupus érythémateux disséminé, les différents types de sclérose, le syndrome de Sjögren primitif (ou syndrome de Gougerot-Sjögren), les polyneuropathies auto-immunes comme la sclérose en plaques, le diabète de type I, les hépatites auto-immunes, la spondylarthrite ankylosante, le syndrome de Reiter, l'arthrite de goutte, la maladie coeliaque, la maladie de Crohn, la thyroïdite chronique d'Hashimoto (hypothyroïdie), la maladie d'Adisson, les hépatites auto-immunes, la maladie de Basedow (hyperthyroïdie), la colite ulcérative, les vascularites comme les vascularites systémiques associées aux ANCA (anticorps anti-cytoplasme des neutrophiles), les cytopénies auto-immunes et autres complications hématologiques de l'adulte et de l'enfant, telles que les thrombopénies auto-immunes aiguës ou chroniques, les anémies hémolytiques auto-immunes, la maladie hémolytique du nouveau-né (MHN), la maladie des agglutinines froides, l'hémophilie acquise auto-immune ; le syndrome de Goodpasture, les néphropathies extra-membraneuses, les affections cutanées bulleuses auto-immunes, la myasthénie réfractaire, les cryoglobulinémies mixtes, le psoriasis, l'arthrite chronique juvénile, les myosites inflammatoires, les dermatomyosites et les affections systémiques auto-immunes de l'enfant incluant le syndrome des antiphospholipides, la maladie des tissus conjonctifs, l'inflammation auto-immune pulmonaire, le syndrome Guillain-Barré, la polyradiculonévrite inflammatoire démyélinisante chronique (PDCI), la thyroïdite auto-immune, le diabète mellitus, le myasthenia gravis, la maladie autoimmune inflammatoire de l'oeil, la neuromyélite optique (maladie de Devic), les sclérodermies, le pemphigus, le diabète par insulinorésistance, la polymyosite, l'anémie de Biermer, la glomérulonéphrite, la maladie de Wegener, la maladie de Horton, la périarthrite noueuse et le syndrome de Churg et Strauss, la maladie de Still, la polychondrite atrophiante, la maladie de Behçet, la gammapathie monoclonale, la granulomatose de Wegener, le lupus, la rectocolite hémorragique, le rhumatisme psoriasique, la sarcoïdose, la colite collagène, la dermatite herpétiforme, la fièvre méditerranéenne familiale, la glomérulonéphrite à dépôts d'IgA, le syndrome myasthénique de Lambert-Eaton, l'ophtalmie sympathique, le syndrome de Fiessinger-Leroy-Reiter et le syndrome uvéo-méningo-encéphalique.

D'autres maladies inflammatoires sont également comprises, comme par exemple le syndrome de détresse respiratoire aiguë (ARDS), l'arthrite septique aiguë, l'arthrite adjuvante, l'encéphalomyélite allergique, la rhinite allergique, la vasculite allergique, l'allergie, l'asthme, l'athérosclérose, l'inflammation chronique due à des infections bactériennes ou virales chroniques, la bronchopneumopathie chronique obstructive (MPOC), les maladies coronariennes, l'encéphalite, les maladies inflammatoires de l'intestin, l'ostéolyse inflammatoire, l'inflammation associée à des réactions d'hypersensibilité aiguë et retardée, l'inflammation associée à des tumeurs, une lésion nerveuse périphérique ou des maladies démyélinisantes, une inflammation associée à un traumatisme des tissus tels que les brûlures et l'ischémie, l'inflammation due à une méningite, le syndrome de défaillance multiviscérale (multiple organ dysfunction syndrome, MODS), la fibrose pulmonaire, la septicémie et le choc septique, le syndrome de Stevens-Johnson, l'arthrite indifférenciée, et les spondylarthropathies indifférenciées.

Dans un mode de réalisation particulier de l'invention, la maladie auto-immune est le purpura thrombotique idiopatique (PTI) et la polyradiculonévrite inflammatoire demyélinisante chronique (PDCI).

La présente invention a également pour objet un procédé de production d'un variant d'un polypeptide parent comprenant un fragment Fc, le dit variant présentant une affinité augmentée pour au moins un des récepteurs du fragment Fc (FcR) choisi parmi les récepteurs FcyRIIIa (CD 16a), FcyRIIa (CD32a), et FcyRIIb (CD32b) par rapport à celle du polypeptide parent, qui comprend une étape de mutation d'au moins un acide aminé, la mutation étant choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

La présente invention a en particulier pour objet un procédé de production d'un variant d'un polypeptide comprenant un fragment Fc, ledit variant présentant une affinité augmentée pour au moins un des récepteurs du fragment Fc (FcR) choisi parmi les récepteurs FcyRIIIa (CD16a), FcyRIIa (CD32a), et FcyRIIb (CD32b), par rapport à celle du polypeptide parent, d'un ratio au moins égal à 2, de préférence supérieur à 5, de préférence supérieur à 10, de préférence supérieur à 15, de préférence supérieur à 20, de préférence supérieur à 25, de préférence supérieur à 30, qui comprend une étape de mutation d'au moins un acide aminé, la mutation étant choisie parmi V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290G, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, Y296W, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

Les séquences décrites dans la présente demande peuvent être résumées comme suit:

| **SEQ ID NO:** | **Protéine** | **Séquence** |
|---|---|---|
| 1 | Région Fc d'IgG1 humaine G1m1,17 (résidus 226-447 selon l'index EU ou équivalent dans Kabat) sans région charnière supérieure N-terminale | CPPCPAPELLGGPSVFLFPPKPKDTLMISRTPEVTCV VVDVSHEDPEVKFNWYVDGVEVHNAKTKPREEQ YNSTYRVVSVLTVLHQDWLNGKEYKCKVSNKAL PAPIEKTISKAKGQPREPQVYTLPPSRDELTKNQVS LTCLVKGFYPSDIAVEWESNGQPENNYKTTPPVLD SDGSFFLYSKLTVDKSRWQQGNVFSCSVMHEALH |

| | | |
|---|---|---|
| | | NHYTQKSLSLSPGK |
| 2 | Région Fc d'IgG2 humaine sans région charnière supérieure N-terminale | CPPCPAPPVAGPSVFLFPPKPKDTLMISRTPEVTCV VVDVSHEDPEVQFNWYVDGVEVHNAKTKPREEQ FNSTFRVVSVLTVVHQDWLNGKEYKCKVSNKGLP APIEKTISKTKGQPREPQVYTLPPSREEMTKNQVSL TCLVKGFYPSDIAVEWESNGQPENNYKTTPPMLDS DGSFFLYSKLTVDKSRWQQGNVFSCSVMHEALHN HYTQKSLSLSPGK |
| 3 | Région Fc d'IgG3 humaine sans région charnière supérieure N-terminale | CPRCPAPELLGGPSVFLFPPKPKDTLMISRTPEVTC VVVDVSHEDPEVQFKWYVDGVEVHNAKTKPREE QYNSTFRVVSVLTVLHQDWLNGKEYKCKVSNKA LPAPIEKTISKTKGQPREPQVYTLPPSREEMTKNQV SLTCLVKGFYPSDIAVEWESSGQPENNYNTTPPML DSDGSFFLYSKLTVDKSRWQQGNIFSCSVMHEALH NRFTQKSLSLSPGK |
| 4 | Région Fc d'IgG4 humaine sans région charnière supérieure N-terminale | CPSCPAPEFLGGPSVFLFPPKPKDTLMISRTPEVTCV VVDVSQEDPEVQFNWYVDGVEVHNAKTKPREEQ FNSTYRVVSVLTVLHQDWLNGKEYKCKVSNKGLP SSIEKTISKAKGQPREPQVYTLPPSQEEMTKNQVSL TCLVKGFYPSDIAVEWESNGQPENNYKTTPPVLDS DGSFFLYSRLTVDKSRWQEGNVFSCSVMHEALHN HYTQKSLSLSLGK |
| 5 | Région Fc d'IgG1 humaine G1m3 sans région charnière supérieure N-terminale | CPPCPAPELLGGPSVFLFPPKPKDTLMISRTPEVTCV VVDVSHEDPEVKFNWYVDGVEVHNAKTKPREEQ YNSTYRVVSVLTVLHQDWLNGKEYKCKVSNKAL PAPIEKTISKAKGQPREPQVYTLPPSREEMTKNQVS LTCLVKGFYPSDIAVEWESNGQPENNYKTTPPVLD SDGSFFLYSKLTVDKSRWQQGNVFSCSVMHEALH NHYTQKSLSLSPGK |
| 6 | Région Fc d'IgG1 humaine G1m1,17 avec région charnière supérieure N-terminale (résidus 216-447 selon l'index EU ou équivalent dans Kabat) | EPKSCDKTHTCPPCPAPELLGGPSVFLFPPKPKDTL MISRTPEVTCVVVDVSHEDPEVKFNWYVDGVEVH NAKTKPREEQYNSTYRVVSVLTVLHQDWLNGKEY KCKVSNKALPAPIEKTISKAKGQPREPQVYTLPPSR DELTKNQVSLTCLVKGFYPSDIAVEWESNGQPENN YKTTPPVLDSDGSFFLYSKLTVDKSRWQQGNVFSC SVMHEALHNHYTQKSLSLSPGK |
| 7 | Région Fc d'IgG2 humaine avec région charnière supérieure N-terminale | ERKCCVECPPCPAPPVAGPSVFLFPPKPKDTLMISR TPEVTCVVVDVSHEDPEVQFNWYVDGVEVHNAK TKPREEQFNSTFRVVSVLTVVHQDWLNGKEYKCK VSNKGLPAPIEKTISKTKGQPREPQVYTLPPSREEM TKNQVSLTCLVKGFYPSDIAVEWESNGQPENNYKT TPPMLDSDGSFFLYSKLTVDKSRWQQGNVFSCSV MHEALHNHYTQKSLSLSPGK |
| 8 | Région Fc d'IgG3 humaine avec région charnière supérieure N-terminale | ELKTPLGDTTHTCPRCPEPKSCDTPPPCPRCPEPKS CDTPPPCPRCPEPKSCDTPPPCPRCPAPELLGGPSVF LFPPKPKDTLMISRTPEVTCVVVDVSHEDPEVQFK WYVDGVEVHNAKTKPREEQYNSTFRVVSVLTV LHQDWLNGKEYKCKVSNKALPAPIEKTISKTKGQP REPQVYTLPPSREEMTKNQVSLTCLVKGFYPSDIA VEWESSGQPENNYNTTPPMLDSDGSFFLYSKLTVD |

| | | |
|---|---|---|
| | | KSRWQQGNIFSCSVMHEALHNRFTQKSLSLSPGK |
| 9 | Région Fc d'IgG4 humaine avec région charnière supérieure N-terminale | ESKYGPPCPSCPAPEFLGGPSVFLFPPKPKDTLMISR TPEVTCVVVDVSQEDPEVQFNWYVDGVEVHNAK TKPREEQFNSTYRVVSVLTVLHQDWLNGKEYKCK VSNKGLPSSIEKTISKAKGQPREPQVYTLPPSQEEM TKNQVSLTCLVKGFYPSDIAVEWESNGQPENNYKT TPPVLDSDGSFFLYSRLTVDKSRWQEGNVFSCSVM HEALHNHYTQKSLSLSLGK |
| 10 | Région Fc d'IgG1 humaine G1m3 avec région charnière supérieure N-terminale | EPKSCDKTHTCPPCPAPELLGGPSVFLFPPKPKDTL MISRTPEVTCVVVDVSHEDPEVKFNWYVDGVEVH NAKTKPREEQYNSTYRVVSVLTVLHQDWLNGKEY KCKVSNKALPAPIEKTISKAKGQPREPQVYTLPPSR EEMTKNQVSLTCLVKGFYPSDIAVEWESNGQPEN NYKTTPPVLDSDGSFFLYSKLTVDKSRWQQGNVFS CSVMHEALHNHYTQKSLSLSPGK |

### EXEMPLES

Les exemples suivants sont donnés en vue d'illustrer divers modes de réalisation de l'invention.

### Exemple 1 : Production de variants Fc selon l'invention par mutagenèse dirigée

### 1. Construction des variants Fc :

Chaque mutation d'intérêt dans le fragment Fc a été insérée indépendamment dans un vecteur d'expression contenant la chaîne lourde anti-CD20 par PCR de chevauchement en utilisant deux ensembles d'amorces adaptées pour intégrer une délétion ou un codon dégénéré (NNN ou NNK) en position ciblée (240 à 243, 258 à 267, 290 à 296, 298 à 305). Les fragments ainsi obtenus par PCR ont été associés et le fragment résultant a été amplifié par PCR en utilisant des protocoles standards. Le produit de PCR a été purifié sur gels d'agarose 1% (w/ v), digéré avec les enzymes de restriction adéquates et cloné dans le vecteur d'expression eucaryote pMGM05-CD20 (pCEP4 InvitroGen), qui contient des sites de clonage pour le fragment Fc (BamHI et NotI) et la chaîne variable VH de l'anticorps anti-CD20. Cette construction entraîne la mutation de deux acides aminés dans le Fc (aa224 et 225, HT changé en GS) et l'ajout de la séquence EFAAA en C-terminal du Fc, mais permet de tester très rapidement un très grand nombre de clones. Dans un premier temps, il a été vérifié que ces mutations ne modifiaient pas la liaison de l'IgG-WT aux différents récepteurs.

L'ADN des clones isolés a été séquencé après PCR sur colonies. Après analyses bio-informatiques, les clones comportant des mutations nouvelles ont été congelés à -80°C en bactérie XL1-Blue et les séquences inclues dans notre base de données.

### 2. Production des IgG variants en cellules HEK293 :

La chaîne légère de l'anti-CD20 a été insérée dans un vecteur pCEP4 identique au vecteur utilisé pour la chaîne lourde, noté pMGM01-CDC20 (pCEP4 InvitroGen). Des cellules HEK293-F Freestyle^{™} (Invitrogen), cultivées dans des plaques à 24 puits, ont été co-transfectées avec les vecteurs pMGM01-CD20 et pMGM05-CD20 (Fc-WT et variants) dans des quantités équimolaires (250 ng/ml) avec un réactif de transfection (1 µl/ml) en utilisant des protocoles standard (Invitrogen). Les cellules ont été cultivées en suspension dans du milieu exempt de sérum pendant 7-9 jours post-transfection et les surnageants (1 ml) contenant des IgG ont été récoltés après centrifugation des cellules à 100 g pendant 10 min.

Les IgG sécrétées dans les surnageants ont été quantifiées en utilisant un test ELISA (FastELISA, R&D biotech).

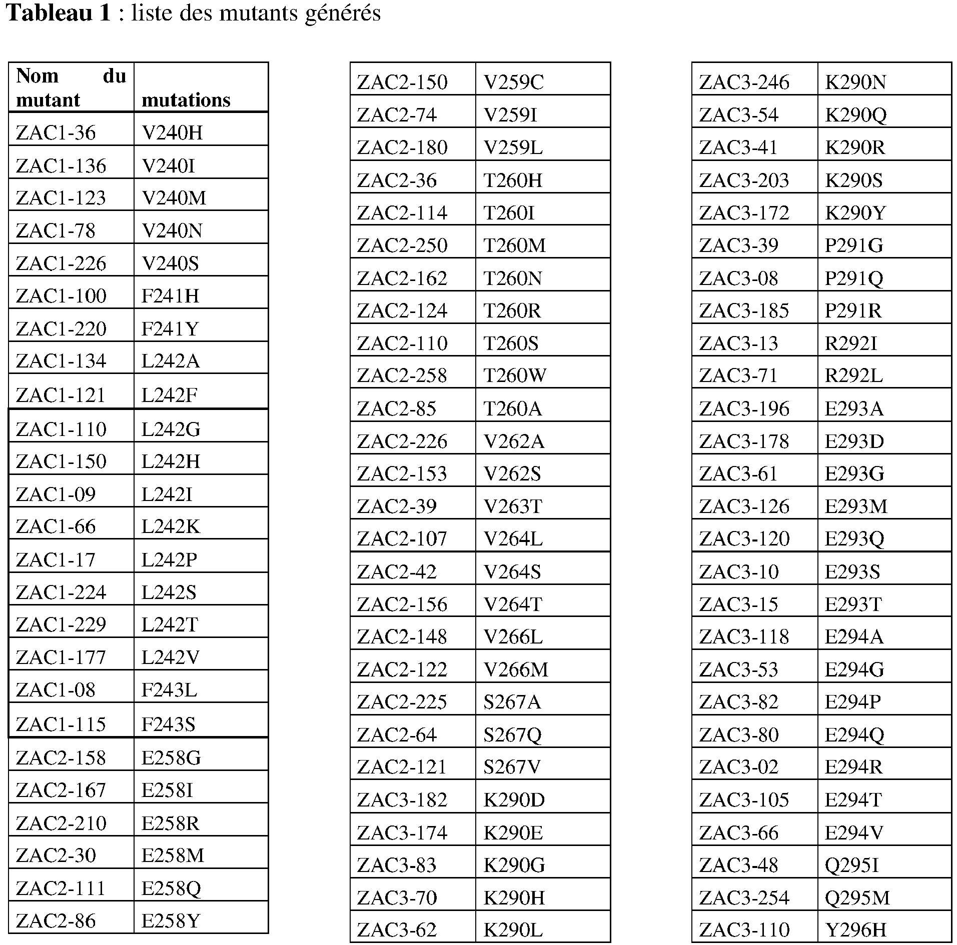

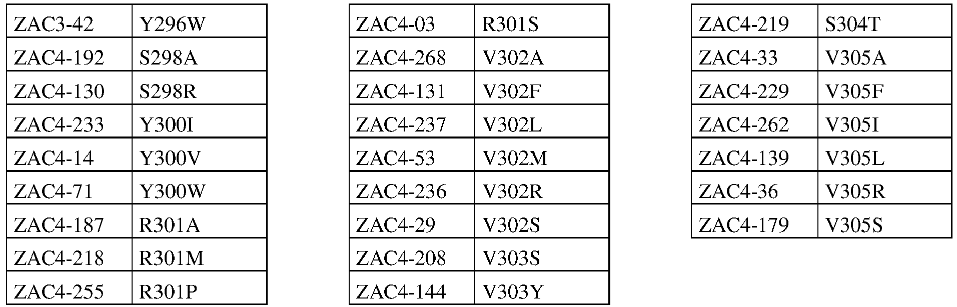

### Exemple 2 : Tests de liaison sur les récepteurs Fc

### 1. Récepteurs Fc recombinants utilisés :

CD16a est un récepteur activateur qui présente un polymorphisme V/F en position 158, sur le site de liaison au Fc. L'affinité est meilleure pour CD16aV. CD16aV est disponible commercialement (R&D system).

CD32a est un récepteur activateur qui présente un polymorphisme H/R en position 131, sur le site de liaison au Fc. L'affinité est meilleure pour CD32aH. CD32aH a été produit par PX'Therapeutics. CD32aR et CD32b sont disponibles commercialement (R&D system).

### 2. Tests ELISA de variants IgG produits dans les surnageants de cellules HEK293-F :

Les variants d'IgG ont été testés pour leur liaison à plusieurs FcR humains et au FcRn par ELISA. Des immunoplaques Maxisorp ont été revêtues avec 0,1 µg CD32aH / puits, ou 0,2 µg CD16aV / puits dans du PBS ou 0,25 µg de FcRn dans du P6 (sodium phosphate 100mM, sodium chloride 50mM pH6.0). Des plaques NiNTA (HisGrab Pierce) ont été revêtues avec 0,05µg CD32aR / puits, ou 0,2µg CD32b / puits dans du PBS. Après le revêtement pendant une nuit à 4°C, les plaques ont été lavées deux fois avec du PBS (ouP6)/0,05% Tween-20 et saturées avec du PBS/4% de BSA (ou P6 4% lait-écrémé) pendant 2 heures à 37°C. En parallèle, les surnageants ont été dilués dans du PBS (ou du P6 pour le test sur FcRn) à une concentration finale de 0,5 µg d'IgG/ml et mélangés avec des F(ab')2 d'IgG HRP de chèvre anti-humaine à la même concentration pendant 2 heures à température ambiante. Les IgG agrégées aux F(ab')2 sont ensuite incubées sous agitation douce pendant 1 heure à 30°C sur les plaques ELISA saturées sans dilution pour CD16aV, CD32aR et CD32b (i.e. IgG à 0,5 µg/ml), diluées dans du PBS à 0,25 µg/ml pour CD32aH et dilués dans du P6 à 0,035 µg/ml pour FcRn. Les plaques sont ensuite révélées avec TMB (Pierce) et l'absorbance est lue à 450 nm.

Grâce à ce test ELISA, les variants construits ont été testés en comparaison avec le type sauvage Fc (Fc-WT) et leur ratio variant/Fc-WT a été calculé, comme indiqué dans le tableau 2 ci-dessous. Les tests ELISA réalisés sur ces variants montrent un ratio supérieur à 2 pour au moins l'un des FcyRs testés.

**Tableau 2 : Tests ELISA de liaison aux récepteurs CD16aV, CD32aH, CD32aR et CD32b. Les résultats sont exprimés en ratio Fc variant selon l'invention/Fc-WT**

| **Nom du variant** | **Mutations** | **Résultats tests ELISA** | | | |
|---|---|---|---|---|---|
| | | **CD16aV** | **CD32aH** | **CD32aR** | **CD32b** |
| ZAC1-36 | V240H | 1,91 | 2,00 | 0,82 | 0,78 |
| ZAC1-136 | V240I | 3,91 | 3,59 | 1,71 | 1,45 |
| ZAC1-123 | V240M | 4,10 | 2,16 | 1,08 | 1,03 |
| ZAC1-78 | V240N | 2,44 | 1,42 | 0,64 | 0,89 |
| ZAC1-226 | V240S | 3,82 | 2,51 | 0,82 | 0,81 |
| ZAC1-100 | F241H | 0,77 | 3,47 | 1,44 | 1,44 |
| ZAC1-220 | F241Y | 1,60 | 5,50 | 1,84 | 1,10 |
| ZAC1-134 | L242A | 3,75 | 3,06 | 1,75 | 1,41 |
| ZAC1-121 | L242F | 5,89 | 5,31 | 1,47 | 1,38 |
| ZAC1-110 | L242G | 4,40 | 2,71 | 1,43 | 1,35 |
| ZAC1-150 | L242H | 1,19 | 2,04 | 1,14 | 1,36 |
| ZAC1-09 | L242I | 5,11 | 4,99 | 1,77 | 1,58 |
| ZAC1-66 | L242K | 7,27 | 2,87 | 3,04 | 1,32 |
| ZAC1-17 | L242P | 1,47 | 2,50 | 1,19 | 1,14 |
| ZAC1-224 | L242S | 3,34 | 2,00 | 0,88 | 0,91 |
| ZAC1-229 | L242T | 1,49 | 2,11 | 1,65 | 1,40 |
| ZAC1-177 | L242V | 2,49 | 6,68 | 1,96 | 1,70 |
| ZAC1-08 | F243L | 5,15 | 2,23 | 1,60 | 1,46 |
| ZAC1-115 | F243S | 2,25 | 1,48 | 1,50 | 1,38 |
| ZAC2-158 | E258G | 1,16 | 6,81 | 1,45 | 1,29 |
| ZAC2-167 | E258I | 2,03 | 6,78 | 1,83 | 1,29 |
| ZAC2-30 | E258M | 1,28 | 4,52 | 1,56 | 1,52 |
| ZAC2-111 | E258Q | 1,88 | 8,75 | 1,55 | 0,85 |
| ZAC2-210 | E258R | 1,66 | 8,17 | 3,70 | 2,60 |
| ZAC2-86 | E258Y | 1,53 | 5,86 | 2,30 | 2,70 |
| ZAC2-150 | V259C | 1,23 | 2,91 | 2,03 | 1,44 |
| ZAC2-74 | V259I | 1,06 | 2,20 | 1,47 | 1,10 |
| ZAC2-180 | V259L | 1,19 | 3,08 | 2,14 | 1,84 |
| ZAC2-85 | T260A | 4,68 | 3,89 | 1,65 | 1,55 |
| ZAC2-36 | T260H | 1,16 | 2,43 | 1,19 | 0,91 |
| ZAC2-114 | T260I | 1,91 | 7,06 | 1,46 | 1,00 |
| ZAC2-250 | T260M | 1,06 | 3,80 | 1,29 | 1,71 |
| ZAC2-162 | T260N | 0,94 | 2,99 | 1,57 | 1,27 |
| ZAC2-124 | T260R | 1,09 | 3,45 | 2,60 | 1,42 |
| ZAC2-110 | T260S | 1,44 | 3,71 | 1,74 | 0,91 |
| ZAC2-258 | T260W | 1,49 | 3,54 | 1,40 | 1,24 |
| ZAC2-226 | V262A | 0,91 | 5,84 | 3,78 | 2,42 |
| ZAC2-153 | V262S | 1,01 | 3,64 | 1,14 | 1,03 |
| ZAC2-39 | V263T | 1,13 | 4,80 | 1,27 | 1,01 |
| ZAC2-107 | V264L | 0,82 | 2,67 | 2,13 | 1,34 |
| ZAC2-42 | V264S | 0,67 | 1,40 | 2,30 | 2,07 |
| ZAC2-156 | V264T | 0,91 | 6,24 | 1,86 | 1,42 |
| ZAC2-148 | V266L | 1,12 | 2,10 | 4,67 | 3,68 |
| ZAC2-122 | V266M | 0,47 | 0,34 | 2,44 | 2,32 |
| ZAC2-225 | S267A | 1,26 | 4,26 | 5,82 | 4,75 |
| ZAC2-64 | S267Q | 0,63 | 0,43 | 2,49 | 3,00 |
| ZAC2-121 | S267V | 0,59 | 0,29 | 2,36 | 2,02 |
| ZAC3-172 | K290Y | 4,79 | 6,72 | 2,16 | 1,00 |
| ZAC3-203 | K290S | 2,28 | 4,70 | 1,76 | 1,21 |
| ZAC3-41 | K290R | 1,12 | 1,58 | 2,15 | 2,39 |
| ZAC3-54 | K290Q | 2,47 | 3,85 | 1,52 | 1,50 |
| ZAC3-246 | K290N | 1,36 | 3,22 | 1,71 | NA |
| ZAC3-62 | K290L | 1,51 | 2,65 | 1,35 | 0,63 |
| ZAC3-70 | K290H | 3,49 | 6,48 | 2,64 | 1,66 |
| ZAC3-83 | K290G | 4,20 | 5,78 | 1,86 | 1,83 |
| ZAC3-174 | K290E | 2,83 | 4,89 | 1,66 | 1,83 |
| ZAC3-182 | K290D | 2,04 | 3,38 | 2,23 | NA |
| ZAC3-185 | P291R | 0,64 | 2,57 | 1,93 | NA |
| ZAC3-08 | P291Q | 1,61 | 2,32 | 0,99 | 0,96 |
| ZAC3-39 | P291G | 1,32 | 2,39 | 1,28 | 1,65 |
| ZAC3-71 | R292L | 1,67 | 2,22 | 0,71 | 0,41 |
| ZAC3-13 | R292I | 0,81 | 2,19 | 0,48 | 0,53 |
| ZAC3-15 | E293T | 0,41 | 1,40 | 2,07 | 1,81 |
| ZAC3-10 | E293S | 1,02 | 2,95 | 1,18 | 1,51 |
| ZAC3-120 | E293Q | 1,78 | 2,17 | 1,49 | NA |
| ZAC3-126 | E293M | 1,32 | 2,42 | 1,81 | NA |
| ZAC3-61 | E293G | 0,48 | 1,37 | 2,44 | 0,89 |
| ZAC3-178 | E293D | 0,79 | 2,68 | 1,80 | NA |
| ZAC3-196 | E293A | 0,89 | 2,99 | 1,91 | NA |
| ZAC3-66 | E294V | 1,00 | 1,91 | 3,03 | 0,93 |
| ZAC3-105 | E294T | 0,80 | 2,34 | 1,13 | NA |
| ZAC3-02 | E294R | 0,71 | 0,90 | 2,09 | 1,58 |
| ZAC3-80 | E294Q | 0,88 | 1,26 | 2,78 | 1,01 |
| ZAC3-82 | E294P | 0,87 | 1,32 | 2,43 | 0,70 |
| ZAC3-53 | E294G | 0,57 | 3,30 | 2,34 | 0,52 |
| ZAC3-118 | E294A | 1,86 | 5,10 | 1,57 | 1,25 |
| ZAC3-254 | Q295M | 1,74 | 2,81 | 1,10 | NA |
| ZAC3-48 | Q2951 | 0,92 | 5,36 | 1,29 | 0,58 |
| ZAC3-42 | Y296W | 3,83 | 1,49 | 1,20 | 1,60 |
| ZAC3-110 | Y296H | 2,17 | 0,86 | 0,98 | NA |
| ZAC4-192 | S298A | 5,53 | 0,24 | 0,48 | 0,51 |
| ZAC4-130 | S298R | 4,37 | 0,66 | 1,09 | 0,66 |
| ZAC4-233 | Y300I | 0,69 | 2,25 | 1,01 | 1,04 |
| ZAC4-14 | Y300V | 0,76 | 2,37 | 0,78 | 2,05 |
| ZAC4-71 | Y300W | 0,87 | 2,34 | 1,14 | 1,06 |
| ZAC4-187 | R301A | 0,81 | 1,25 | 2,06 | 2,11 |
| ZAC4-218 | R301M | 0,95 | 1,22 | 1,78 | 2,07 |
| ZAC4-255 | R301P | 0,63 | 4,64 | 0,06 | 0,24 |
| ZAC4-03 | R301S | 1,29 | 2,42 | 1,16 | 1,06 |
| ZAC4-268 | V302A | 1,00 | 2,38 | 1,86 | 3,09 |
| ZAC4-131 | V302F | 0,76 | 2,82 | 1,06 | 0,71 |
| ZAC4-237 | V302L | 0,46 | 0,31 | 3,15 | 4,69 |
| ZAC4-53 | V302M | 0,63 | 1,50 | 2,25 | 1,56 |
| ZAC4-236 | V302R | 0,45 | 0,38 | 5,08 | 10,56 |
| ZAC4-29 | V302S | 1,10 | 2,51 | 1,87 | 1,71 |
| ZAC4-208 | V303S | 0,96 | 1,39 | 1,64 | 2,27 |
| ZAC4-144 | V303Y | 1,00 | 3,50 | 2,10 | 1,03 |
| ZAC4-219 | S304T | 0,98 | 1,82 | 2,14 | 2,34 |
| ZAC4-33 | V305A | 0,55 | 1,00 | 2,20 | 2,04 |
| ZAC4-229 | V305F | 1,34 | 1,11 | 2,15 | 2,40 |
| ZAC4-262 | V305I | 1,22 | 1,26 | 1,87 | 2,22 |
| ZAC4-139 | V305L | 1,19 | 2,72 | 1,87 | 0,82 |
| ZAC4-36 | V305R | 1,33 | 3,50 | 1,71 | 2,42 |
| ZAC4-179 | V305S | 1,29 | 1,65 | 2,03 | 1,66 |

### Exemple 3 : Tests de liaison de variants IgG purifiés sur les récepteurs Fc

### 1. Tests ELISA de variants IgG purifiés :

Les variants produits dans les surnageants de cellules HEK293-F tel que décrit dans l'exemple 1 ont été purifiés par un procédé classique de chromatographie d'affinité sur protéine A.

Les variants construits et purifiés ont été testés en comparaison avec le type sauvage Fc (Fc-WT), selon les protocoles ci-dessous. Leur ratio variant/Fc-WT a été calculé, comme indiqué dans le tableau 3 ci-dessous.

### Liaison au FcRn humain (hFcRn) :

Des immunoplaques Maxisorp ont été revêtues avec le FcRn en tampon phosphate à pH6 (250ng par puits) pendant une nuit à 4°C (100µl/puits). Après saturation des plaques pendant 2 heures en tampon phosphate pH6 et lait écrémé 5%, les solutions d'IgG variants ont été ajoutées dans chaque puits à des concentrations croissantes (de 0,00488 à 10µg/ml) pendant 1h à 37°C puis ont été mises en contact avec des F(ab')2 d'IgG HRP de chèvre anti-Fab humaine pendant 1h à 37°C. Les IgG liées ont été détectées après révélation au TMB par mesure de l'absorbance à 450 nm.

### Liaison au CD64 humain (hCD64) :

Des immunoplaques Maxisorp ont été revêtues avec le récepteur humain CD64 (100ng/puits) pendant une nuit à 4°C (100µl/puits). Après saturation des plaques pendant 2 heures en tampon PBS et BSA 4%, les solutions d'IgG variants ont été ajoutées dans chaque puits à des concentrations croissantes (0,03125 à 1µg/ml) pendant 1h à 37°C puis ont été mises en contact avec des F(ab')2 d'IgG HRP de chèvre anti-CK humaine pendant 1h à 37°C. Les IgG liées ont été détectées après révélation au TMB par mesure de l'absorbance à 450 nm.

### Liaison aux FcyRs humains :

Des immunoplaques Maxisorp ont été revêtues avec 50ng hCD32aH/puits, 200ng hCD16aF/puits ou 75ng hCD16aV/puits dans du PBS. Des plaques chélatantes au nickel immobilisantes (Hisgrab Pierce) ont été revêtues avec 50ng hCD32aR/puits ou 100ng hCD32b/puits dans du PBS. Après le revêtement pendant une nuit à 4°C, les plaques ont été lavées deux fois avec du PBS/0,05% Tween-20 et saturées avec du PBS/4% de BSA pendant 2 heures à 37°C. En parallèle, les solutions d'IgG variants ont été diluées dans du PBS à une concentration finale de 1 µg d'IgG/ml et mélangées avec des F(ab')2 d'IgG HRP de chèvre anti-Fab à la même concentration pendant 2 heures à température ambiante. Les IgG agrégées aux F(ab')2 ont ensuite été incubées sous agitation douce pendant 1 heure à 30°C sur les plaques ELISA saturées à différentes dilutions en PBS. Les plaques ont ensuite été révélées avec TMB et l'absorbance lue à 450 nm.

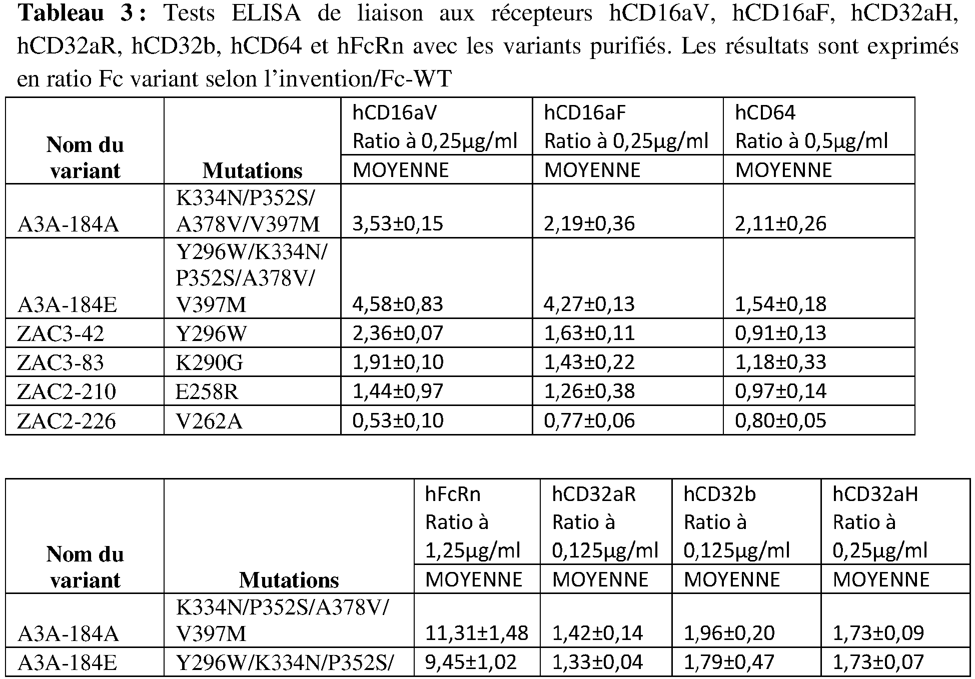

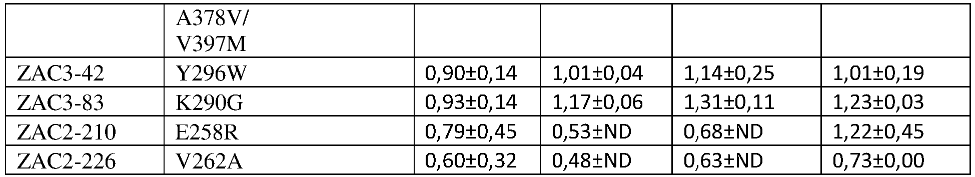

### 2. Tests de liaison sur OctetO (technologie BLI « Bio-Layer Interferometry », Pall) :

On utilise des Biosensors Anti Penta-HIS (HIS 1K). On immobilise le récepteur hCD16aV (R&D Systems) dilué à 1 µg/ml en Kinetics Buffer, soit 44 nM. Les IgG variants ont été testés à 1000, 500, 250, 125, 62.5, 31.25, 15 et 0 nM en Kinetics Buffer. L'analyse des KD a été réalisée avec un modèle d'association 1:1.

Les résultats figurent en tableau 4.

**Tableau 4 : Tests de liaison sur OctetO avec les variants purifiés**

| **Nom du variant** | **Mutations** | **KD hCD16aV [BLI] (nM)** | **Ratio KD WT/variant** |
|---|---|---|---|
| A3A-184A | K334N/P352S/A378V/V397M | 47 | 12,39 |
| A3A-184E | Y296W/K334N/P352S/A378V/ V397M | 80 | 7,25 |
| ZAC3-42 | Y296W | 288 | 2,02 |
| ZAC3-83 | K290G | 326 | 1,78 |
| ZAC2-210 | E258R | 411 | 1,41 |
| ZAC1-08 | F243L | 472 | 1,23 |
| ZAC2-85 | T260A | 472 | 1,23 |
| ZAC1-123 | V240M | 491 | 1,18 |
| ZAC1-121 | L242F | 512 | 1,13 |
| ZAC2-226 | V262A | 518 | 1,12 |
| ZAC1-110 | L242G | 541 | 1,07 |
| Fc-WT | SEQ ID NO : 1 | 580 | 1,00 |

### Exemple 3 : Production d'IgG variants additionnels en cellules HEK293

Des combinaisons de mutants comprenant au moins une mutation i) selon l'invention ont été produites à partir d'un fragment Fc comprenant les mutations de départ N315D/A330V/N361D/A378V/N434Y (mutant T5A-74), ou V259I/N315D/N434Y (mutant C6A_74) ou N315D/N361D/A378V/N434Y (mutant T5A_74A) ou K334N/P352S/V397M/A378V (mutant A3A_184A). Elles sont indiquées en tableau 5.

**Tableau 5 : variants additionnels générés dans le cadre de l'invention**

| Nom | Variant de départ | Mutation i) selon l'invention ajoutée | 1 Liste des mutations combinées 1 |
|---|---|---|---|
| T5A-74I | T5A-74 | T260A | T260A/N315D/A330V/N361D/A378V/N434Y |
| T5A-74J | T5A-74 | E258I | E258I/N315D/A330V/N361D/A378V/N434Y |
| T5A-74K | T5A-74 | K290Y | K290Y/N315D/A330V/N361D/A378V/N434Y |
| T5A-74L | T5A-74 | E294A | E294A/N315D/A330V/N361D/A378V/N434Y |
| T5A-74M | T5A-74 | Y296W | Y296W/N315D/A330V/N361D/A378V/N434Y |
| C6A_74W | C6A_74 | Y296W | Y296W/V259I/N315D/N434Y |
| C6A_74G | C6A_74 | K290G | K290G/V259I/N315D/N434Y |
| T5A-74MA | T5A_74A | Y296W | Y296W/N315D/N361D/A378V/N434Y |
| T5A_74AG | T5A_74A | K290G | K290G/N315D/N361D/A378V/N434Y |
| A3A_184AY | A3A_184A | N434Y | N434Y/K334N/P352S/V397M/A378V |
| A3A_184E | A3A_184A | Y296W | Y296W/K334N/P352S/V397M/A378V |
| A3A_184AG | A3A_184A | K290G | K290G/K334N/P352S/V397M/A378V |

## Revendications

1. Variant d'un polypeptide parent comprenant un fragment Fc, le dit variant présentant une affinité augmentée pour au moins un des récepteurs du fragment Fc (FcR) choisi parmi les récepteurs FcyRIIIa (CD16a), FcyRIIa (CD32a), et FcyRIIb (CD32b) par rapport à celle du polypeptide parent, **caractérisé en ce qu'**il comprend au moins une mutation choisie parmi K290G, Y296W, V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

2. Variant selon la revendication **1,** présentant une affinité augmentée pour au moins un des récepteurs du fragment Fc (FcR) choisi parmi les récepteurs FcyRIIIa (CD16a), FcyRIIa (CD32a), et FcyRIIb (CD32b), par rapport à celle du polypeptide parent, d'un ratio au moins égal à 2, de préférence supérieur à 5, de préférence supérieur à 10, de préférence supérieur à 15, de préférence supérieur à 20, de préférence supérieur à 25, de préférence supérieur à 30.

3. Variant selon la revendication **1 ou 2,** présentant une affinité augmentée pour le récepteur FcyRIIIa (CD16a) et comprenant au moins une mutation choisie parmi K290G, Y296W, S298A, S298R, F243L, F243S, L242A, L242F, L242G, L242I, L242K, L242S, L242V, V240I, V240M, V240N, V240S, E258I, T260A, K290D, K290E, K290H, K290Q, K290S, K290Y et Y296H dudit fragment Fc;
la numérotation étant celle de l'index EU ou équivalent dans Kabat.

4. Variant selon la revendication **1 ou 2,** présentant une affinité augmentée pour le récepteur FcyRIIa (CD32a), et comprenant au moins une mutation choisie parmi K290G, F241H, F241Y, F243L, L242A, L242F, L242G, L242H, L242I, L242K, L242P, L242S, L242T, L242V, V240H, V240I, V240M, V240S, E258G, E258I, E258R, E258M, E258Q, E258Y, S267A, S267Q, S267V, T260A, T260H, T260I, T260M, T260N, T260R, T260S, T260W, V259C, V259I, V259L, V262A, V262S, V263T, V264L, V264S, V264T, V266L, V266M, E293A, E293D, E293G, E293M, E293Q, E293S, E293T, E294A, E294G, E294P, E294Q, E294R, E294T, E294V, K290D, K290E, K290H, K290L, K290N, K290Q, K290R, K290S, K290Y, P291G, P291Q, P291R, Q295I, Q295M, R292I, R292L, R301A, R301P, R301S, S304T, V302A, V302F, V302L, V302M, V302R, V302S, V303Y, V305A, V305F, V305L, V305R, V305S, Y300I, Y300V et Y300W ; la numérotation étant celle de l'index EU ou équivalent dans Kabat.

5. Variant selon la revendication **1 ou 2,** présentant une affinité augmentée pour le récepteur FcyRIIb (CD32b) et comprenant au moins une mutation choisie parmi E258R, E258Y, V262A, S267A, S267Q, S267V, V264S, V266L, V266M, K290R, R301A, R301M, S304T, V302A, V302L, V302R, V303S, V305A, V305F, V305I, V305R, et Y300V dudit fragment Fc ; la numérotation étant celle de l'index EU ou équivalent dans Kabat.

6. Variant selon l'une des revendications **1 à 5**, **caractérisé en ce que** le fragment Fc du polypeptide parent comprend déjà au moins :
(ii) une mutation choisie parmi 378V, 378T, 434Y et 434S; et
(iii) au moins une mutation choisie parmi 226G, P228L, P228R, 230S, 230T, 230L, 241L, 264E, 307P, 315D, 330V, 362R, 378V, 378T, 389T, 389K, 434Y et 434S,
la numérotation étant celle de l'index EU ou équivalent dans Kabat et avec la condition que les mutations (ii) et (iii) n'aient pas lieu sur les mêmes acides aminés.

7. Variant selon l'une des revendications **1 à 5**, **caractérisé en ce que** le fragment Fc du polypeptide parent comprend déjà au moins :
(iv) une mutation choisie parmi 307N, 326E, 326T, 334N, 334R, 352L, 378V, 378T, 394P, 396L, 397M, et 421T ; et
(v) au moins une mutation choisie parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N,
la numérotation étant celle de l'index EU ou équivalent dans Kabat et avec la condition que les mutations (iv) et (v) n'aient pas lieu sur les mêmes acides aminés.

8. Variant selon l'une des revendications **1 à 7, caractérisé en ce qu'**il comprend de 1 à 20 mutations dudit fragment Fc, de préférence de 1 à 10 mutations.

9. Variant selon l'une des revendications **1 à 8, caractérisé en ce que** le polypeptide parent comprend un fragment Fc parent qui est un fragment Fc humain, de préférence un fragment Fc d'une IgG1 humaine ou d'une IgG2 humaine.

10. Variant selon l'une des revendications **1 à 9, caractérisé en ce qu'**il est choisi parmi un fragment Fc isolé, une séquence dérivée d'un fragment Fc isolé, un anticorps et une protéine de fusion comprenant un fragment Fc.

11. Variant d'un polypeptide parent selon l'une des revendications **1 à 10, caractérisé en ce qu'**il est un anticorps.

12. Variant selon la revendication **1 à 11,** dirigé contre un antigène choisi parmi un antigène tumoral, un antigène viral, un antigène bactérien, un antigène fongique, une toxine, une cytokine membranaire ou circulante, un récepteur membranaire.

13. Variant selon l'une des revendications **1 à 12**, pour son utilisation comme médicament.

14. Composition pharmaceutique comprenant (1) un variant selon l'une des revendications **1 à 12**, et (2) au moins un excipient pharmaceutiquement acceptable.

15. Procédé de production d'un variant d'un polypeptide comprenant un fragment Fc, le dit variant présentant une affinité augmentée pour au moins un des récepteurs du fragment Fc (FcR) choisi parmi les récepteurs FcyRIIIa (CD16a), FcyRIIa (CD32a), et FcyRIIb (CD32b) par rapport à celle du polypeptide parent, **caractérisé en ce qu'**il comprend une étape de mutation d'au moins un acide aminé, la mutation étant choisie parmi K290G, Y296W, V240H, V240I, V240M, V240N, V240S, F241H, F241Y, L242A, L242F, L242G, L242H, L242I, L242K, L242P, L242S, L242T, L242V, F243L, F243S, E258G, E258I, E258R, E258M, E258Q, E258Y, V259C, V259I, V259L, T260A, T260H, T260I, T260M, T260N, T260R, T260S, T260W, V262A, V262S, V263T, V264L, V264S, V264T, V266L, V266M, S267A, S267Q, S267V, K290D, K290E, K290H, K290L, K290N, K290Q, K290R, K290S, K290Y, P291G, P291Q, P291R, R292I, R292L, E293A, E293D, E293G, E293M, E293Q, E293S, E293T, E294A, E294G, E294P, E294Q, E294R, E294T, E294V, Q295I, Q295M, Y296H, S298A, S298R, Y300I, Y300V, Y300W, R301A, R301M, R301P, R301S, V302A, V302F, V302L, V302M, V302R, V302S, V303S, V303Y, S304T, V305A, V305F, V305I, V305L, V305R et V305S dudit fragment Fc; la numérotation étant celle de l'index EU ou équivalent dans Kabat.

16. Procédé de production d'un variant d'un polypeptide comprenant un fragment Fc selon la revendication **15,** ledit variant présentant une affinité augmentée pour au moins un des récepteurs du fragment Fc (FcR) choisi parmi les récepteurs FcyRIIIa (CD16a), FcyRIIa (CD32a), et FcyRIIb (CD32b), par rapport à celle du polypeptide parent, d'un ratio au moins égal à 2, de préférence supérieur à 5, de préférence supérieur à 10, de préférence supérieur à 15, de préférence supérieur à 20, de préférence supérieur à 25, de préférence supérieur à 30.
